# EUROPEAN PATENT APPLICATION

(11) **EP 4 122 510 A1**
(43) Date of publication of application: **25.01.2023**
(21) Application number: 20925617.1
(22) Date of filing: 15.10.2020
(51) Int. Cl.: A61M 5/168

(54) **FLOW RATE CONTROL REGULATOR FOR FLUID INFUSION**

(30) Priority: 17.03.2020 KR 20200032764
(71) Applicant: MEDI LINE ACTIVE KOREA CO., LTD., Gyeonggi-do 15455 (KR)
(72) Inventor: KANG, Dae Won, Seoul 04202 (KR)
(74) Representative: Caspary, Karsten
(86) International application number: PCT/KR2020/014072
(87) International publication number: WO 2021/187707

(57) **Abstract**

The present invention relates to a flow rate regulator for intravenous injection, which is capable of preventing an operating roller from rotating, in a state where a flow rate of an intravenous fluid is adjusted by the operating roller, to keep the flow rate of the intravenous fluid from being varied, the flow rate regulator including: a body having a space portion penetratedly formed thereinto in an up and down direction to fittingly connect an infusion tube thereto and a roller connection hole formed on the front surface thereof; the operating roller fitted to the roller connection hole of the body, moving up and down along the roller connection hole, and pressurizing the infusion tube thereagainst to allow the flow rate of the intravenous fluid to be regulated; and a roller holder connected to the body to which the operating roller is fitted and supporting the operating roller thereagainst to prevent the operating roller from arbitrarily moving.

## Description

### Technical Field

The present invention relates to a flow rate regulator for intravenous (IV) injection, and more specifically, to a flow rate regulator for IV injection that is capable of preventing an operating roller from rotating, in a state where a flow rate of an IV fluid is adjusted by the operating roller, to thus keep the flow rate of the IV fluid from being varied.

### Background Art

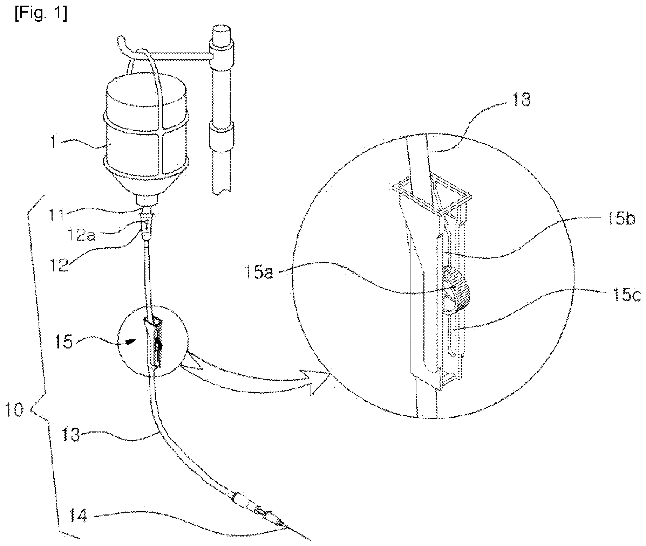

As shown in FIG. 1, an IV injection system for injecting an IV fluid into the vein includes an IV fluid bottle 1 in which the IV fluid is stored, an insertion needle 11 inserted into a sealing stopper of the IV fluid bottle 1 to allow the IV fluid from flowing out from the IV fluid bottle 1, a drip chamber 12 fixed to the underside of the insertion needle 11 to allow the IV fluid from fall down therefrom in the form of drops (gtt) 12a, an injection needle 14 inserted into the vein, a tube 13 serving as an IV fluid injection path for connecting the drip chamber 12 and the injection needle 14, and an IV fluid flow regulator 15 mounted on the intermediate portion of the tube 13 to adjust the flow rate of the IV fluid.

Generally, the insertion needle 11, the drip chamber 12, the tube 13, the injection needle 14, and the IV fluid flow regulator 15 are made as a single set, and such a set is called an infusion set 10.

After the IV fluid is injected into a patient' body in a state where the infusion set 10 is connected to the IV fluid bottle 1, if it is desired to continuously inject the IV fluid into the same patient, only the IV fluid bottle 1 in which the IV fluid is all consumed is exchanged with new one.

Further, the drops 12a falling down in the drip chamber 12 are the IV fluid drops like water drops, and so as to provide the drops 12a having given volumes, the insertion needle 11 and the drip chamber 12 are made. For example, if it is desired to make 20 drops per 1 cc of the IV fluid, the volume of one drop becomes 1/20 cc, and if it is desired to make 60 drops per 1 cc of the IV fluid, the volume of one drop becomes 1/60 cc.

If a cycle in which the drops fall from the drip chamber 12 is measured, the flow rate of the IV fluid injected through the infusion set 10 is calculated.

So as to inject the IV fluid into the patient's body, further, the flow rate of the IV fluid injected is prescribed according to the type of IV fluid, the types of drugs added to the IV fluid, the patient's condition, the type of the patient's disease, and the like, and through the IV fluid flow regulator 15, accordingly, the IV fluid is injected at the prescribed flow rate.

In this case, if the flow rate of IV fluid injected does not conform to the prescribed flow rate, medical accidents may occur, and therefore, it is very important to accurately regulate the flow rate of IV fluid. The IV fluid flow regulator 15 has an operating part 15a for adjusting the sectional area of the IV fluid passing through the tube 13, and through the operation of the operating part 15a, the flow rate of IV fluid injected is controlled.

The IV fluid flow regulator 15 as shown in FIG. 1 is called a roller clamp type flow regulator, and accordingly, it has the operating part 15a having a roller type. In specific, the tube 13 fittedly passes through a hole 15b penetrated up and down into the IV fluid flow regulator 15, and the operating part 15a for pressurizing the tube 13 moves up and down along a long concave groove 15c formed on the IV fluid flow regulator 15.

In this case, the hole 15b becomes narrow in width as it goes toward the bottom thereof, and as the operating part 15a moves down, it more strongly pressurizes the tube 13. As the operating part 15a moves, the flow rate of IV fluid is measured at the moving position, and the movement of the operating part 15a is stopped at a desired flow rate, thereby finishing the control of the flow rate of IV fluid injected.

However, the roller clamp type IV fluid flow regulator 15 as shown in FIG. 1 is configured to observe the drip chamber 12 whenever the roller type operating part 15a moves so as to measure the flow rate of IV fluid, so that such inconvenient measurements may be repeatedly needed and a degree of accuracy in the flow rate control may be deteriorated.

Further, the conventional fluid flow regulator is configured to move the roller type operating part up and down to control the flow rate of IV fluid injected, but after the operation of the operating part, no locking means for locking the operating part is provided so that if the operating part operates due to the patient's motions or mistakes, it may move unexpectedly to cause the flow rate of IV fluid to be varied from the set value, which undesirably leads medical accidents.

### Disclosure

### Technical Problem

Accordingly, the present invention has been made in view of the above-mentioned problems occurring in the related art, and it is an object of the present invention to provide a flow rate regulator for IV injection that is capable of allowing a roller holder for supporting an operating roller to be connected to a body to which an infusion tube is fitted to prevent the operating roller for regulating an amount of an intravenous fluid from moving at a regulated position thereof, and of allowing flow rate scales indicated on the body to discharge the intravenous fluid by set amount safely and accurately, thereby improving product performance, safety of use, and reliability.

### Technical Solution

To accomplish the above-mentioned objects, according to the present invention, there is provided a flow rate regulator for intravenous injection, including: a body having a space portion penetratedly formed thereinto in an up and down direction to fittingly connect an infusion tube thereto and a roller connection hole formed on the front surface thereof; an operating roller fitted to the roller connection hole of the body, moving up and down along the roller connection hole, and pressurizing the infusion tube thereagainst to allow the flow rate of an intravenous fluid to be regulated; and a roller holder connected to the body to which the operating roller is fitted and supporting the operating roller thereagainst to prevent the operating roller from arbitrarily moving.

The body may include flow rate scales indicated thereon to check the flow rate of the intravenous fluid discharged through the infusion tube according to the movement of the operating roller.

The roller holder may include: a fitting part coupled to the roller connection hole; and a movement prevention part disposed at the front surface of the fitting part and coming into close contact with one side surface of the operating roller to prevent the operating roller from moving.

The fitting part may include: fitting pieces fitted to the roller connection hole and moving up and down along the roller connection hole; and locking pieces protruding outward from the ends of the fitting pieces locking pieces and locked onto the inside of the roller connection hole to prevent the roller holder from escaping from the roller connection hole.

The fitting pieces may come into close contact with both inner walls of the roller connection hole, respectively.

The movement prevention part may have the shape of c with a support hole formed at the inside thereof to allow the outer periphery of the operating roller to come into fittedly close contact therewith.

The movement prevention part may have a plurality of moving protrusions formed on the front surface thereof to easily move the roller holder along the roller connection hole so that the roller holder comes into close contact with the operating roller.

The roller holder may include: a connection part fitted to the roller connection hole and connected to the operating roller; and locking parts connected to the connection part and locked onto the outer surfaces of the body to prevent the roller holder from moving so that the operating roller becomes locked.

The connection part may include: a moving plate coming into close contact with the top surface of the body and having a fitting hole formed thereinto to fit an upward protruding portion of the operating roller; and connection stands disposed on both sides of the underside of the moving plate and having fixing grooves formed on the undersides thereof, fitted to the roller connection hole and the rotary shafts of the operating roller simultaneously, and moving the roller holder together with the operating roller.

The locking parts may be located at both sides of the connection part, respectively and have locking hooks disposed on the undersides thereof and locked onto the locking protrusions formed on the outer surfaces of the body to allow the roller holder is locked onto the body.

The locking parts may be flexibly connected to the connection part to rotate up and down gently with respect to the connection part.

### Advantageous Effects

According to the present invention, the flow rate regulator for intravenous injection is configured to have the roller holder adapted to temporarily stop the movement of the operating roller along the body to which the infusion tube is fitted so that the operating roller for regulating the amount of the intravenous fluid is locked onto the body by means of the roller holder to prevent the arbitrary movement from the position, and even though the operating roller operates due to the patient's motions or mistakes, it does not easily move to allow the set amount of intravenous fluid to be consistently discharged, thereby preventing medical accidents from occurring and optimizing the efficiency of the product.

### Brief Description of Drawings

FIG. 1 is a perspective view showing an installation state of a conventional infusion set and an IV fluid flow regulator mounted on the infusion set.
FIG. 2 is an exploded perspective view showing a flow rate regulator for IV injection according to an embodiment of the present invention.
FIG. 3 is a perspective view showing the flow rate regulator for IV injection according to the present invention.
FIG. 4 is a front view showing the flow rate regulator for IV injection according to the present invention.
FIG. 5 is a cross sectional view showing the flow rate regulator for IV injection according to the present invention.
FIG. 6 is a perspective view showing a roller holder of the flow rate regulator for IV injection according to the present invention.
FIG. 7 is a front view showing the roller holder of FIG. 6.
FIG. 8 is an exploded perspective view showing a flow rate regulator for IV injection according to another embodiment of the present invention.
FIG. 9 is a perspective view showing a state before the flow rate regulator for IV injection of FIG. 8 is locked.
FIG. 10 is a perspective view showing a state in which the flow rate regulator for IV injection of FIG. 8 is locked.
FIG. 11 is a perspective view showing a state in which the roller holder of the flow rate regulator for IV injection of FIG. 8 is coupled to an operating roller.
FIG. 12 is a front view showing the roller holder of the flow rate regulator for IV injection of FIG. 8.
FIG. 13 is a side view showing the roller holder of the flow rate regulator for IV injection of FIG. 8.
FIG. 14 is a cross sectional view showing the flow rate regulator for IV injection of FIG. 8.

### <Explanations of reference numerals in the drawings>

| | |
|---|---|
| 100: Body | 110: Space portion |
| 120: Roller connection hole | 130: Locking projection |
| 140: Flow rate scale | 200: Operating roller |
| 210: Rotary shaft | 300, 301: Roller holder |
| 310: Fitting part | 311: Fitting piece |
| 312: Locking piece | 320: Movement prevention part |
| 321: Support hole | 322: Moving protrusion |
| 330: Connection part | 331: Moving plate |
| 331a: Fitting hole | 332: Connection stand |
| 332a: Fixing groove | 340: Locking part |
| 341: Locking hook | |

### Mode for Invention

The present invention may be modified in various ways and may have several exemplary embodiments. Specific exemplary embodiments of the present invention are illustrated in the drawings and described in detail in the detailed description. However, this does not limit the invention within specific embodiments and it should be understood that the invention covers all the modifications, equivalents, and replacements within the idea and technical scope of the invention.

In the description, the thicknesses of the lines or the sizes of the components shown in the drawing may be magnified for the clarity and convenience of the description. Further, the terms as will be discussed later are defined in accordance with the functions of the present invention, but may be varied under the intention or regulation of a user or operator. Therefore, they should be defined on the basis of the whole scope of the present invention.

A term 'and/or' includes a combination of a plurality of relevant and described items or any one of a plurality of related and described items. When it is said that one element is described as being "connected" or "coupled" to the other element, one element may be directly connected or coupled to the other element, but it should be understood that another element may be present between the two elements. In this application, terms, such as "comprise", "include", or "have", are intended to designate those characteristics, numbers, steps, operations, elements, or parts which are described in the specification, or any combination of them that exist, and it should be understood that they do not preclude the possibility of the existence or possible addition of one or more additional characteristics, numbers, steps, operations, elements, or parts, or combinations thereof.

In the description, when it is said that a layer, a region, a pattern, or a structure is located "on" or "under" another layer, region, pattern, or structure, it means that one layer may come into contact with another layer as well as yet another layer may exist between the two layers. The relation between the layers is explained with reference to the drawings.

Hereinafter, exemplary embodiments of the present invention will be described in detail with reference to the accompanying drawings.

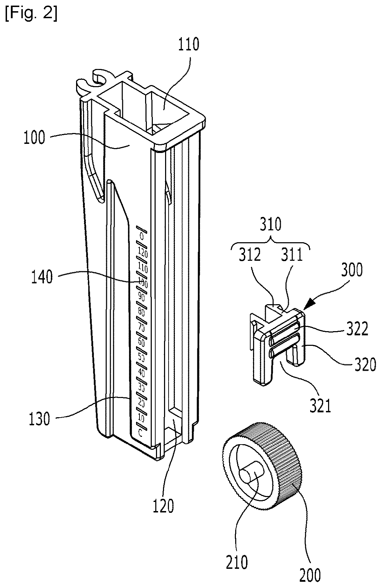

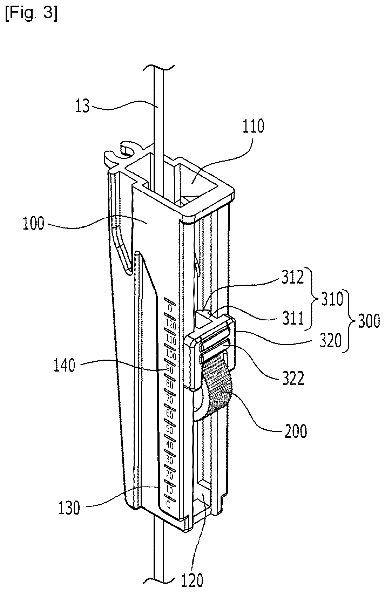

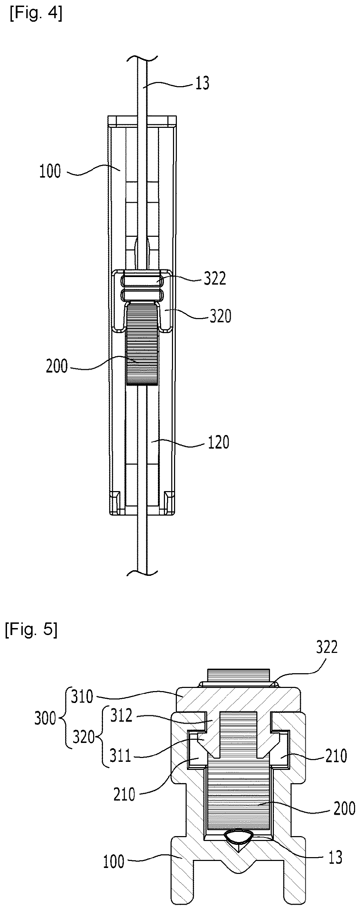

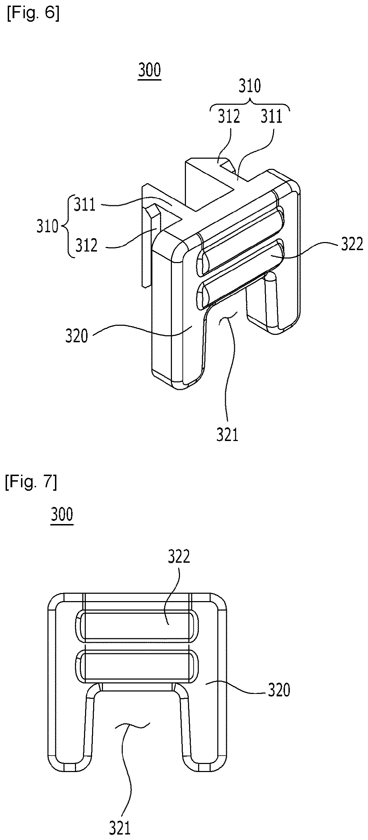

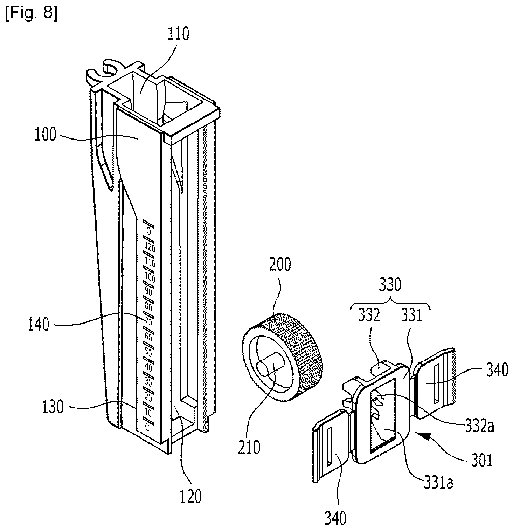

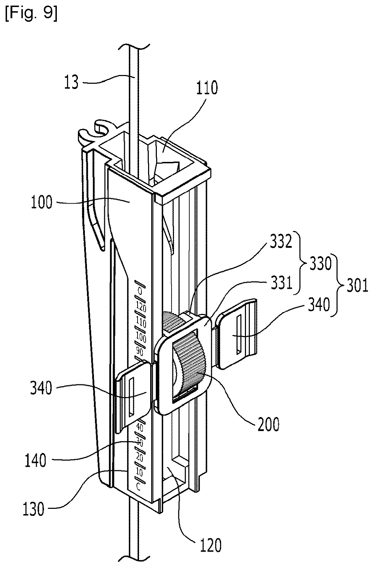

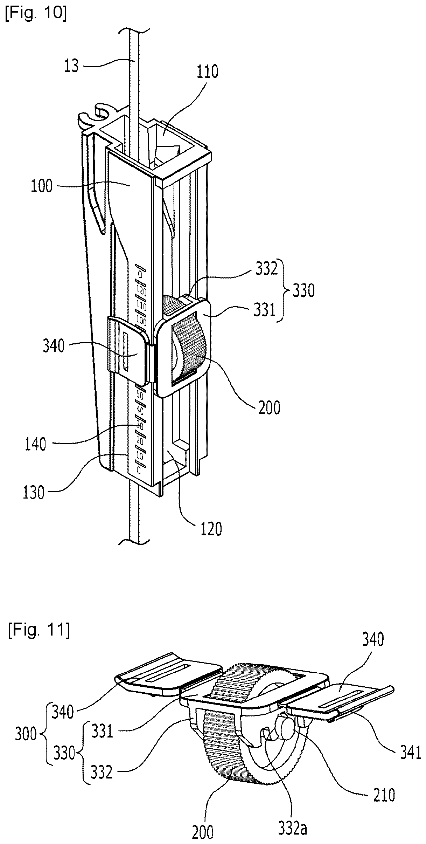

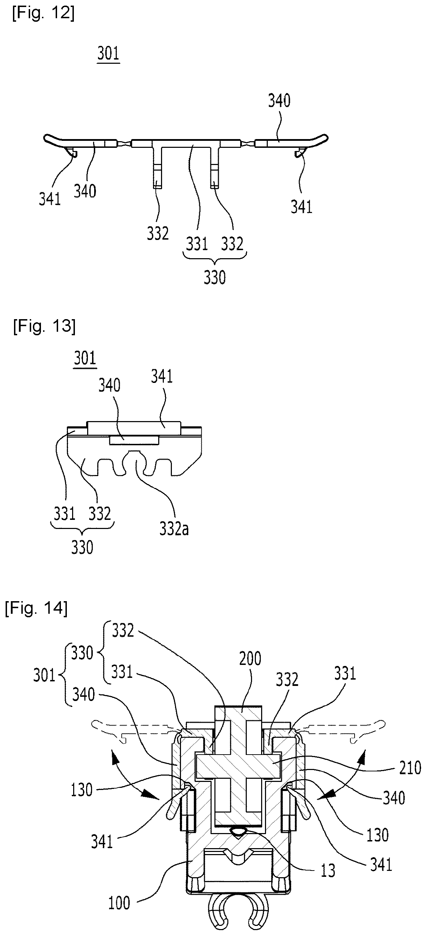

FIG. 2 is an exploded perspective view showing a flow rate regulator for IV injection according to an embodiment of the present invention, FIG. 3 is a perspective view showing the flow rate regulator for IV injection according to the present invention, FIG. 4 is a front view showing the flow rate regulator for IV injection according to the present invention, FIG. 5 is a cross sectional view showing the flow rate regulator for IV injection according to the present invention, FIG. 6 is a perspective view showing a roller holder of the flow rate regulator for IV injection according to the present invention, FIG. 7 is a front view showing the roller holder of FIG. 6, FIG. 8 is an exploded perspective view showing a flow rate regulator for IV injection according to another embodiment of the present invention, FIG. 9 is a perspective view showing a state before the flow rate regulator for IV injection of FIG. 8 is locked, FIG. 10 is a perspective view showing a state in which the flow rate regulator for IV injection of FIG. 8 is locked, FIG. 11 is a perspective view showing a state in which the roller holder of the flow rate regulator for IV injection of FIG. 8 is coupled to an operating roller, FIG. 12 is a front view showing the roller holder of the flow rate regulator for IV injection of FIG. 8, FIG. 13 is a side view showing the roller holder of the flow rate regulator for IV injection of FIG. 8, and FIG. 14 is a cross sectional view showing the flow rate regulator for IV injection of FIG. 8.

As shown in FIGs. 2 to 7, a flow rate regulator for IV injection according to an embodiment of the present invention largely includes a body 100, an operating roller 200, and a roller holder 300.

The body 100 has a space portion 110 penetratedly formed thereinto in an up and down direction to fittingly connect an infusion tube 13 thereto and a roller connection hole 120 formed on the front surface thereof to connect the operating roller 200 thereto.

In this case, the space portion 110 becomes inclined toward the bottom thereof to thus regulate the depth pressurized of the infusion tube 13 according to the movement of the operating roller 200.

Further, the body 100 has locking projections 130 protruding from the outer surfaces thereof.

The body 100 includes flow rate scales 140 indicated on the side surface thereof to check the flow rate of an intravenous fluid discharged through the infusion tube 13 according to the movement of the operating roller 200.

The operating roller 200 is fitted to the roller connection hole 120 of the body 100 and pressurizes the infusion tube 13 according to upward and downward movements thereof to allow the flow rate of the intravenous fluid to be regulated.

Further, the operating roller 200 has rotary shafts 210 disposed on both side surfaces thereof and fitted to the side surfaces of the roller connection hole 120 so that the operating roller 200 moves up and down along the roller connection hole 120.

The roller holder 300 is fitted to the roller connection hole 120 of the body 100 and comes into close contact with one side of the operating roller 200 to support the operating roller 200 thereagainst so that the operating roller 200 is prevented from arbitrarily moving.

As shown in FIGs. 2, 6 and 7, the roller holder 300 includes a fitting part 310 and a movement prevention part 320.

The fitting part 310 is fitted to the roller connection hole 120 of the body 100 and moves up and down.

The fitting part 310 has fitting pieces 311 fitted to the roller connection hole 120 and moving up and down along the roller connection hole 120.

Further, the fitting pieces 311 come into close contact with both inner walls of the roller connection hole 120, respectively.

That is, the fitting pieces 311 fitted to the roller connection hole 120 come into close contact with both inner walls of the roller connection hole 120 so that unless the roller holder 300 is controlled by a manager, it does not easily move up and down along the roller connection hole 120.

Further, the fitting part 310 has locking pieces 312 protruding outward from the ends of the fitting pieces 311 and thus locked onto the inside of the roller connection hole 120 so that the roller holder 300 is prevented from escaping from the roller connection hole 120.

Further, the locking pieces 312 protrude outward from the ends of the fitting pieces 311 and are easily fitted to the inside of the roller connection hole 120, and in this case, they have the shape of Z, so that they do not easily escape from the roller connection hole 120.

The movement prevention part 320 is disposed at the front surface of the fitting part 310 and comes into close contact with one side surface of the operating roller 200 to prevent the operating roller 200 from moving.

Further, as shown in FIG. 4, the movement prevention part 320 is disposed above the operating roller 200.

That is, as the operating roller 200 moves down, the force of the operating roller 200 pressurizing the infusion tube 12 becomes gradually strong to decrease the amount of the intravenous fluid discharged, and accordingly, the operating roller 200 does not move down easily but moves up. As a result, the roller holder 300 is located above the operating roller 200.

The movement prevention part 320 has the shape of ⊏ with a support hole 321 formed at the inside thereof to fit one side outer periphery of the operating roller 200 thereto and thus prevent the operating roller 200 from moving.

Further, the movement prevention part 320 has a plurality of moving protrusions 322 formed on the front surface thereof to easily move the roller holder 300 along the roller connection hole 120 so that the roller holder 300 comes into close contact with the operating roller 200 to prevent the operating roller 200 from moving.

In this case, the moving protrusions 322 protrude upward from the front surface of the movement prevention part 320.

As shown in FIGs. 8 to 14, a flow rate regulator for IV injection according to another embodiment of the present invention is configured to have a roller holder 301 with a connection part 330 and locking parts 340.

The connection part 330 is fitted to the roller connection hole 120, connected to the operating roller 200, and moves together with the operating roller 200.

The connection part 330 includes a moving plate 331 and connection stands 332.

The moving plate 331 comes into close contact with the top surface of the body 100 and has a fitting hole 331a formed thereinto to fit an upward protruding portion of the operating roller 200 fitted to the roller connection hole 120 thereto.

That is, as the moving plate 331 comes into close contact with the body 100, the operating roller 200 is fitted to the inside of the fitting hole 331a, and accordingly, the moving plate 331 is movable according to the movement of the operating roller 200.

The connection stands 332 are disposed vertically on both sides of the underside of the moving plate 331 and fitted to the roller connection hole 120 so that they are fitted to the rotary shafts 210 of the operating roller 200, respectively, to move the roller holder 301 to move together with the operating roller 200.

Further, the connection stands 332 have fixing grooves 332a formed on the undersides thereof to fit the rotary shafts 210 of the operating roller 200 thereto.

That is, the connection stands 332 are fitted between the roller connection hole 120 and the operating roller 200, and accordingly, the rotary shafts 210 are fitted to the fixing grooves 332a, so that the operating roller 200 and the roller holder 301 move simultaneously.

The locking parts 340 are connected to the connection part 330 and locked onto the locking projections 130 formed on the outer surfaces of the body 100 according to the rotation of the operating roller 200, thereby preventing the roller holder 301 from moving to thus allow the operating roller 200 to be locked.

In this case, the locking parts 340 are located at both sides of the connection part 330, respectively.

The locking parts 340 have locking hooks 341 disposed on the undersides thereof to be locked onto the locking protrusions 130 formed on the outer surfaces of the body 100 so that the roller holder 301 is locked onto the body 100.

In this case, the locking hooks 341 are provided by incising portions of the locking parts 340 and then bending the incised portions downward.

Further, the locking parts 340 are flexibly connected to the connection part 330 and thus rotate up and down gently with respect to the connection part 330 to allow the roller holder 301 to be locked onto the body 100 or released from the locked state.

Now, an explanation of an operating state of the flow rate regulator for IV injection according to the present invention will be given below.

The operating roller 200 is connected to the roller connection hole 120 of the body 100 having the infusion tube 13 fitted thereto, and next, the roller holder 300 or 301 is fitted to the roller connection hole 120 to which the operating roller 200 is fitted, so that the operating roller 200 can be prevented from moving along the roller connection hole 120 by means of the roller holder 300 or 301.

Next, the roller holder 300 or 301 fitted to the roller connection hole 120 serves to support the operating roller 200 or lock it onto the body 100, and accordingly, the operating roller 200, which is adjusted to a set amount of intravenous fluid, does not arbitrarily move unless it is controlled by the manager, so that the set amount of intravenous fluid is constantly discharged.

Further, the flow rate scales 140 are indicated on the side surface of the body 100, and accordingly, the amount of intravenous fluid varied according to the movement of the operating roller 200 can be accurately checked by the manager, not by his or her eye measurement, so that it is possible to discharge the amount of intravenous fluid according to prescription, thereby maximizing the efficiency of a product.

In addition, the roller holder 300 or 301 is separately coupled to the roller connection hole 120 of the body 100 in a simple manner, and if necessary, accordingly, they can be detachably attached easily to the body 100.

While the present invention has been described with reference to the particular illustrative embodiments, it is not to be restricted by the embodiments but only by the appended claims. It is to be appreciated that those skilled in the art can change or modify the embodiments without departing from the scope and spirit of the present invention.

## Claims

1. A flow rate regulator for intravenous injection, comprising:
a body having a space portion penetratedly formed thereinto in an up and down direction to fittingly connect an infusion tube thereto and a roller connection hole formed on the front surface thereof;
an operating roller fitted to the roller connection hole of the body, moving up and down along the roller connection hole, and pressurizing the infusion tube thereagainst to allow the flow rate of an intravenous fluid to be regulated; and
a roller holder connected to the body to which the operating roller is fitted and supporting the operating roller thereagainst to prevent the operating roller from arbitrarily moving.

2. The flow rate regulator according to claim 1, wherein the body comprises flow rate scales indicated thereon to check the flow rate of the intravenous fluid discharged through the infusion tube according to the movement of the operating roller.

3. The flow rate regulator according to claim 1, wherein the roller holder comprises:
a fitting part coupled to the roller connection hole; and
a movement prevention part disposed at the front surface of the fitting part and coming into close contact with one side surface of the operating roller to prevent the operating roller from moving.

4. The flow rate regulator according to claim 3, wherein the fitting part comprises:
fitting pieces fitted to the roller connection hole and moving up and down along the roller connection hole; and
locking pieces protruding outward from the ends of the fitting pieces and locked onto the inside of the roller connection hole to prevent the roller holder from escaping from the roller connection hole.

5. The flow rate regulator according to claim 4, wherein the fitting pieces come into close contact with both inner walls of the roller connection hole, respectively.

6. The flow rate regulator according to claim 3, wherein the movement prevention part has the shape of ⊏ with a support hole formed at the inside thereof to allow the outer periphery of the operating roller to come into fittedly close contact therewith.

7. The flow rate regulator according to claim 3, wherein the movement prevention part has a plurality of moving protrusions formed on the front surface thereof to easily move the roller holder along the roller connection hole so that the roller holder comes into close contact with the operating roller.

8. The flow rate regulator according to claim 1, wherein the roller holder comprises:
a connection part fitted to the roller connection hole and connected to the operating roller; and
locking parts connected to the connection part and locked onto the outer surfaces of the body to prevent the roller holder from moving so that the operating roller becomes locked.

9. The flow rate regulator according to claim 8, wherein the connection part comprises:
a moving plate coming into close contact with the top surface of the body and having a fitting hole formed thereinto to fit an upward protruding portion of the operating roller; and
connection stands disposed on both sides of the underside of the moving plate and having fixing grooves formed on the undersides thereof, fitted to the roller connection hole and the rotary shafts of the operating roller simultaneously, and moving the roller holder together with the operating roller.

10. The flow rate regulator according to claim 8, wherein the locking parts are located at both sides of the connection part, respectively and have locking hooks disposed on the undersides thereof and locked onto the locking protrusions formed on the outer surfaces of the body to allow the roller holder is locked onto the body.

11. The flow rate regulator according to claim 8, wherein the locking parts are flexibly connected to the connection part to rotate up and down gently with respect to the connection part.
